(19) Europäisches Patentamt
European Patent Office
Office européen des brevets

(11) Publication number: **0 002 259**
**B1**

# (12) EUROPEAN PATENT SPECIFICATION

(45) Date of publication of patent specification: **03.10.84**

(21) Application number: **78101491.5**

(22) Date of filing: **01.12.78**

(51) Int. Cl.³: **C 07 D 233/86,**
C 07 D 403/00, A 61 K 31/415
// (C07D403/00, 233/86, 257/04)

## (54) Hydantoin derivatives and salts thereof, their synthesis and pharmaceutical formulations.

(30) Priority: **01.12.77 GB 5009377**

(43) Date of publication of application:
**13.06.79 Bulletin 79/12**

(45) Publication of the grant of the patent:
**03.10.84 Bulletin 84/40**

(84) Designated Contracting States:
**BE CH DE FR GB IT LU NL SE**

(56) References cited:
**FR-A-2 362 839**
**FR-A-2 374 309**
**GB-A- 817 745**
**GB-A-1 346 279**
**GB-A-1 529 275**

(73) Proprietor: **THE WELLCOME FOUNDATION LIMITED**
**183-193 Euston Road**
**London NW1 2BP (GB)**

(72) Inventor: **Whittaker, Norman**
**37 Brabourne Rise**
**Beckenham Kent (GB)**
Inventor: **Caldwell, Albert Gordon**
**119 Gates Green Road**
**West Wickham Kent (GB)**

(74) Representative: **Berg, Wilhelm, Dr. et al**
**Dr. Berg, Dipl.-Ing. Stapf, Dipl.-Ing. Schwabe, Dr. Dr. Sandmair Patentanwälte Postfach 860245**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 002 259 B1**

## Description

This invention relates to heterocyclic compounds, processes for their preparation, compositions containing the heterocyclic compounds for use in medicine.

Thiohydantoin derivatives, defined hereinbelow in formula (I), have been found to have pharmacological properties related to those of natural prostaglandins, as demonstrated by their ability to mimic or antagonise the physiological effects of the natural prostaglandins in various biological preparations. In particular, certain compounds of formula (I) have been found to be potent mimetics of the anti-platelet aggregatory properties of prostaglandin $E_1$.

The subject matter of the present invention is a compound of formula (I)

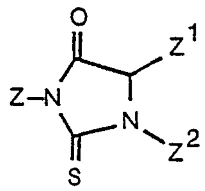

(I)

in which

Z is hydrogen or alkyl having from 1 to 6 carbon atoms;

one of $Z^1$ and $Z^2$ is represented by the group $—CH_2—X—X^1—X^2$ wherein X is phenylene, $—C \equiv C—$, *cis* or *trans* $—CH = CH—$ or $—CH_2—CQ_2—$ in which each Q is independently selected from hydrogen and alkyl having from 1 to 6 carbon atoms or the two Q's together form an alkylene radical having four, five or six carbon atoms;

$X^1$ is a covalent bond or a straight or branched alkylene chain having from 1 to 6 carbon atoms, optionally having one methylene group replaced by an oxa (—O—) or thia (—S—) group, provided that at least one carbon atom separates such an oxa or thia group from a $—C \equiv C—$, $—CH = CH—$ or —CO— group; and

$X^2$ is selected from 5-tetrazolyl, carboxyl, carbamoyl, hydroxymethyl, protected-hydroxymethyl and alkoxycarbonyl, wherein the alkyl group has from 1 to 6 carbon atoms and the other of $Z^1$ and $Z^2$ is represented by the group $—Y—Y^1—Y^2—Y^3$, wherein Y is $—CR_2—CR_2—$ in which each R is independently selected from hydrogen and methyl;

$Y^1$ is carbonyl, methylene, methylene substituted by hydroxy or protected-hydroxy, or methylene substituted by hydroxy or protected-hydroxy and by alkyl;

Provided always that when $X^1$ is or includes an oxa or thia group, then $Y^1$ is carbonyl, methylene substituted by hydroxy or protected-hydroxy, or methylene substituted by hydroxy or protected-hydroxy and by alkyl having from 1 to 6 carbon atoms;

$Y^2$ is a covalent bond or a straight or branched alkylene chain having from 1 to 7 carbon atoms, optionally substituted on the carbon atom adjacent $Y^1$ by one or two groups each of which may be alkyl having from 1 to 6 carbon atoms or a cyclic radical, $Y^3$ is hydrogen, hydroxy, protected-hydroxy, alkoxy having from 1 to 7 carbon atoms, a cyclic radical, phenyl, benzyl, phenoxy or benzyloxy, wherein each of phenyl, benzyl, phenoxy and benzyloxy may be substituted in the benzene ring by one or more groups selected from hydroxy, protected-hydroxy, halo, nitro, amino, acylamino having from 1 to 6 carbon atoms, alkenyl having from 2 to 4 carbon atoms, alkoxy having from 1 to 6 carbon atoms, phenyl and alkyl having from 1 to 6 carbon atoms which may itself be substituted by one or more halo groups;

the cyclic radical in the definitions of $Y^2$ and $Y^3$ above being a radical derived by removal of a ring hydrogen atom from a monocyclic or polycyclic compound, other than benzene or naphthalene, having from 3 to 12 ring atoms selected from carbon, nitrogen, oxygen, and sulphur which compound may be saturated or unsaturated and may be further substituted by one or more alkyl or fluoro groups, said alkyl groups having each from 1 to 6 carbon atoms;

or $Y^2$ and $Y^3$ together form an alkyl group, having from 1 to 7 carbon atoms, at least one hydrogen of which is replaced by fluoro;

or Y is a covalent bond $—CH_2—$, or $—CH_2—CH_2—$ and $Y^1$, $Y^2$ and $Y^3$ taken together form a cycloalkyl or bicycloalkyl group substituted by a hydroxy or protected-hydroxy group;

or a salt thereof.

In formula (I), the term cyclic radical means the monovalent radical derived by loss of a ring hydrogen atom from a monocyclic or polycyclic compound other than benzene or naphthalene having from 3 to 12 ring atoms selected from carbon, nitrogen, oxygen, and sulphur, which compound may be saturated or unsaturated and may be further substituted by one or more alkyl groups, but excluding phenyl. Such cyclic radicals include cycloalkyl having 3 to 10 carbon atoms such as cyclopropyl, cyclopentyl, cyclohexyl and cyclooctyl, bicycloalkyl having 4 to 10 carbon atoms such as adamantyl or norbornanyl (bicyclo[2,2,3]heptyl), spiroalkanyl having 5 to 12 carbon atoms such as 2-spiro[3,3]heptyl, 1-spiro[4,4]nonane and 8-spiro[4,5]decane, cycloalkenyl having 4 to 10 carbon atoms such as 4-cyclopentene, heterocyclic radicals such as tetrahydrofuranyl and tetrahydropyranyl and heteroaryl radicals such as thienyl, furyl, pyridyl, pyrimidyl, thiazolyl, imidazolyl and diazepinyl. Included in the term cyclic radical are there wherein one or more hydrogen atoms are replaced by fluoro.

Unless otherwise stated, in formula (I) and other formulae in this specification alkyl moieties are selected from methyl, ethyl, propyl, butyl, pentyl and hexyl, including all isomers thereof, i.e. having from 1 to 6 carbon atoms for example in the definitions of $Y^1$ and $Y^2$ the alkyl groups are preferably methyl; and the alkyl moiety of alkoxycarbonyl is preferably methyl or ethyl. Similarly alkenyl groups have 2 to 4 carbon atoms for example vinyl.

In a compound of formula (I) the bonding of the divalent phenylene group may be *ortho*, *meta* or *para*, and the oxa or thia group is preferably adjacent the phenylene or when X is other than phenylene then $X^1$ may be $-CH_2-O-CH_2-$.

Included in the meaning of compounds of formula (I) are the salts corresponding to the carboxylic acids and tetrazoles when $X^2$ is carboxyl or tetrazolyl respectively, and the salts which may also be formed when Z is hydrogen. Particularly valuable salts for human medical purposes are those having a pharmaceutically acceptable cation such as ammonium or that of an alkali metal eg. sodium and potassium, an alkaline earth metal eg. calcium and magnesium, or an organic base, particularly an amine such as ethanolamine. Salts having non-pharmaceutically acceptable cations are included within the ambit of this invention as useful intermediates to pharmaceutically acceptable salts, or the acids or esters of formula (I).

Except when there is clear indication to the contrary, formula (I) and other formulae in the specification embrace all stereoisomers represented therein. In particular such formulae include the enantiomeric forms, such mixtures as are designated racemates, and diastereoisomers.

Preferably Z is hydrogen (or a salt thereof) and $Z^1$ is $-CH_2-X-X^1-X^2-$ as defined for formula (I).

The compound of formula (I) wherein Z is hydrogen or alkyl having 1 to 4 carbon atoms, for example methyl or butyl;

one of $Z^1$ and $Z^2$ is $-CH_2-X-X^1-X^2-$ wherein X and $X^1$ taken together form alkylene of 3 to 7 in particular 5 carbon atoms (including alkylene in which X is *cis* or *trans* $-CH=CH-$, i.e. X and $X^1$ form an alkylene radical) and $X^2$ is alkoxycarbonyl, carboxyl or a salt thereof;

and the other of $Z^1$ and $Z^2$ is $-Y-Y^1-Y^2-Y^3-$ wherein Y, $Y^1$ and $Y^2$ are as hereinbefore defined and $Y^3$ is hydrogen, phenyl, benzyl, or cycloalkyl of 4 to 7 carbon atoms;

have particularly interesting prostaglandin-related properties. Within this definition are included the subclass wherein Z is hydrogen or salt thereof and $Z^1$ is $-CH_2-X-X^1-X^2$ as defined.

The compounds of formula (I) may be prepared by adapting methods known from the preparation of analogous compounds.

In particular, a convenient synthesis is by reacting a compound of formula (II)

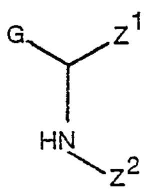

(II)

wherein $Z^1$ and $Z^2$ have the same meaning as in formula (I) and G is a carboxyl group, alkoxycarbonyl, carbamoyl or nitrile group with thiocyanic or alkyl *iso* thiocyanic acid or an alkyl*iso*thiocyanate as appropriate, depending on whether Z is hydrogen or alkyl respectively.

When thiocyanic acid is used, the acid is conveniently produced *in situ* by the use of an alkali metal thiocyanate, eg. potassium thiocyanate and ammonium thiocyanate, and an acid which may be present as an acid addition salt of the compound of formula (II) of a free acid of formula (II) wherein either or both of G and $X^2$ is carboxy. Alternatively an equivalent amount of mineral acid or an organic acid may be added to the reaction medium. The reaction may proceed in the absence of a solvent but preferably an inert solvent is used which is preferably polar such as water or a mixture of water with acetone, dimethylformamide, dimethylsulphoxide or a lower alkanol such as ethanol or it may be nonpolar such as a hydrocarbon, an ether or halogenated hydrocarbon such as chloroform. Where desired, for example if no solvent is used, the reaction may be promoted by heating the reactants.

Similar reaction conditions may be used when alkyl *iso*-thio cyanate is used except that it is unnecessary to provide an equivalent amount of acid, as an acid addition salt or otherwise, in the reactants.

Instead of using a thiocyanate or isothiocyanate, a compound of formula (II) may be reacted with thiourea, nitrothiourea or an *N*-alkylthiourea as appropriate. A solvent is not essential but if desired an inert solvent such as one mentioned above may be used, and the reaction is preferably effected at an elevated temperature, for example from 100° to 125°C but temperatures up to 150°C may be employed.

An intermediate of formula (II) may be conveniently prepared by reaction of a compound of formula (III) with a compound of formula (IV).

(III) 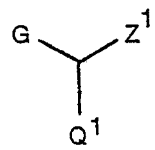 $Q^2 - Z^2$ (IV)

wherein G, $Z^1$ and $Z^2$ are as defined in formula (III), one of $Q^1$ and $Q^2$ is amino and the other is halogeno, preferably bromo. The reaction may be carried out by heating in the absence of solvent or in the presence of an inert solvent such as ethanol.

The intermediates of formula (II) wherein $Z^2$ is —Y—$Y^1$—$Y^2$—$Y^3$ when $Y^1$ is carbonyl may also be prepared by reaction of an amine of formula (III) wherein $Q^1$ is amino with an unsaturated ketone of formula (V).

$$CR_2 = CH.CO.Y^2.Y^3 \qquad (V)$$

wherein R, $Y^2$ and $Y^3$ have the same meaning as in formula (I): the reaction being effected in the presence or absence of an inert solvent, and at room temperature or optionally with heating.

Tetrazoles of formula (I) ($X^2$ being 5-tetrazolyl) may be prepared from corresponding compounds wherein the group —$X^2$ is replaced by

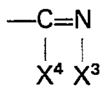

wherein $X^3$ and $X^4$ together form a bond (nitrile), $X^3$ is hydrogen or alkyl and $X^4$ is alkoxy (imidoester), alkylthio (imidothioester), —NH—$NH_2$ (amidrazone), or amino (amidine) or $X^3$ is hydroxy and $X^4$ is amino (amidoxime). The reaction is preferably carried out in a polar aprotic liquid medium such as dimethylformamide using a salt of hydrazoic acid eg. sodium azide. However, when $X^2$ is replaced by an amidine or amidrazone, a suitable reagent is nitrous acid. If an amidine is reacted with nitrous acid then reduction of the intermediate nitrosation product, with or without prior isolation, using for example sodium amalgam is required to give the corresponding tetrazole. The precursor to the tetrazole may be obtained by well known methods, for example the nitrile may be obtained by dehydration of the corresponding amide.

The alcohols of formula (I) wherein $X^2$ is hydroxymethylene may also be obtained by reduction with an appropriate reducing agent of the corresponding acid, ester, acid halide, acid anhydride or aldehyde. The appropriate reducing agent will depend on the particular substrate, but reagent which may be used is sodium in ethanol. In particular a carboxylic acid may for example be converted to a corresponding mixed anhydride with ethylchloroformate in the presence of a base such as triethylamine, and subsequently reduced to the alcohol using sodium borohydride. Similarly an ester may be reduced to the alcohol using di-*iso*-butyl aluminium hydride in an inert solvent such as ether or hydrocarbon such as hexane or benzene. Such alcohols may also be prepared by catalytic hydrogenation.

Alternatively hydroxyl group-containing compounds of formula (I), especially the alcohols wherein $X^2$ is hydroxymethylene may be prepared by hydrolysis or a corresponding halide with an appropriate reagent. For this purpose a hydroxide may be used for example an aqueous alkali or a suspension of silver oxide in water.

In the synthesis of compounds formula of (I) having a hydroxyl group in a side chain it may be desirable to protect this hydroxyl group during the course of the reaction. This may be readily effected in known manner using a protecting group such as acyl, aroyl, tetrahydropyran-2-yl, 1-ethoxyethyl or aralkyl, for example benzyl.

Removal of protecting groups may be carried out by appropriate methods known to those skilled in the art: for example an acyl group may be removed by acid or base hydrolysis, and a benzyl group by reductive cleavage.

Furthermore a ketone of formula (I) wherein $Y^1$ is carbonyl may be converted to the corresponding secondary alcohol by reduction with a suitable reducing agent, such as sodium borohydride. Also, an alcohol of formula (I) wherein $Y^1$ is —CH.OH— may be oxidised to the corresponding ketone using Jones' reagent, acid dichromate or any other suitable reagent.

Similarly where the compounds of formula (I) have a $C \equiv C$ or $CH = CH$ or —$CH = CQ$— link these may be converted by conventional hydrogenation techniques, for example using a Lindlar type or Adams catalyst, to the corresponding ethylenic or saturated compounds as appropriate.

The compounds of formula (I) have an asymmetric 5-carbon atom in the hydantoin ring and a further asymmetric centre is present in those compounds wherein $Y^1$ includes a hydroxyl group. Such alcohols therefore exist as four isomers which are separable by thin layer chromotography or high performance liquid chromatography into two diastereomers, each of which is a racemic mixture of two isomers. On separation of the diastereomers the less polar diastereomers being preferred, one

diasetereomer may be converted to a mixture of the four isomers by treatment with a base, such as an alkali metal hydroxide, and subsequently re-separated to provide two diastereomers. Repeated use of this technique enables the effective conversion of one diastereomer to the other; this may be desirable when one diastereomer has a biological activity preferred to the other.

The corresponding alcohols of formula (II) or (III) also exist in four isomeric forms. If desired, these may be separated into two epimers and subsequent cyclisation to a compound of formula (I) retains the stereochemical configuration.

Other asymmetric centres can be present: for example if only one Q in formula (I) is other than hydrogen.

In all of the foregoing chemical procedures it is of course evident that the choice of reactant will be dictated in part by the functional groups present in the substrate, and where necessary reactants having an appropriate selectivity of action must be used.

The compounds of formula (I) are of value in having pharmacological properties related to those of natural prostaglandins that is, the hydantoins mimic or antagonise the biological effects of members of the prostaglandin (PG) 'A', 'B', 'C', 'D', 'E' and 'F' series. For example, compounds of formula (I) have been found to mimic the anti-aggregatory effect of $PGE_4$ on blood platelets, and to antagonise the contraction induced by $PGE_2$ or $PGF_2$ on smooth muscle taken from the rat stomach, rat colon, chick rectum and guinea pig trachea. In general, antagonistic properties, as opposed to mimetic, have been observed when using larger doses of the hydantoins. The pharmacological profile, by which is meant the relative activities, mimetic or antagonistic, compared with the natural prostaglandins, will of course vary depending on the specific hydantoin under consideration.

By reason of their prostaglandin-related properties, the compounds of formula (I) are useful in the pharmacological characterisation and differentiation of the biological activities of the natural prostaglandins and their 'receptors'. The further understanding of the physiological role of the prostaglandins is of course valuable in the search for new and improved therapeutic substances.

The compounds of formula (I) are also of value as therapeutic agents. In particular compounds such as those described hereinbefore as having a potent anti-aggregatory effect on blood platelets are useful when it is desired to inhibit platelet aggregation or to reduce the adhesive character of platelets, and may be used to treat or prevent thrombo-embolic disorders, e.g. the formation of thrombi in mammals, including man. For example, the compounds are useful in the treatment and prevention of myocardial infarcts, to treat and prevent thrombosis, to promote patency of vascular grafts following surgery, and to treat complications of arteriosclerosis and conditions such as atherosclerosis, blood clotting defects due to lipermia, and other clinical conditions in which the underlying aetiology is associated with lipid imbalance or hyperlipidemia. A further use for such compounds is as an additive to blood and other fluids which are used in artificial extra-corporeal circulation and perfusion of isolated body portions.

A group of compounds which have been found particularly valuable as inhibitors of platelet aggregation are those of formula (I) wherein Z is hydrogen; $Z^1$ is carboxyalkylene wherein the alkylene moiety has 3 to 9 carbon atoms; and $Z^2$ is a group $—(CH_2)_2.CH.OH.Y^2.Y^3$ wherein $Y^2$ is a bond or branched alkylene having a tertiary carbon atom adjacent the hydroxy-substituted carbon and $Y^3$ is as defined in formula (I). Within this group of compounds, those wherein $Z^1$ is carboxyhexyl or carboxyhexenyl and $Y^3$ is cycloalkyl having 4 to 7 carbon atoms have been found especially active.

It has also been found that compounds of formula (I) cause relaxation of vascular smooth muscle in a similar way as do members of the prostaglandin 'A' and 'E' series. Compounds relaxing vascular smooth muscle are capable of inducing vasodilation and therefore have antihypertensive properties and are useful in lowering blood pressure in mammals, including man, and may be used alone or in combination with a $\beta$-adrenoceptor blocking agent or another antihypertensive substance for the treatment of all grades of hypertension including essential, malignant and secondary hypertension.

Thiohydantoins of formula (I) also mimic the effect of $PGE_1$ of antagonising histamine induced broncho-constriction, and such compounds may be used in the treatment or prophylaxis of bronchial asthma and bronchitis by alleviating the bronchoconstriction associated with this condition.

Thiohydantoins of formula (I), which inhibit pentagastrin-induced gastric acid secretion and reduce the formation of aspirin-induced gastric lesions in rats are useful in reducing excessive gastric secretion, reducing and avoiding gastro-intestinal ulcer formation and accelerating the healing of such ulcers already present in the gastro-intestinal tract whether such ulcers arise spontaneously or as a component of polyglandular adenoma syndromes.

Intravenous infusions of certain compounds of formula (I) to dogs will increase the urine volume indicating a potential utility for such compounds as diuretic agents, the uses of which include the treatment of oedema for example oedema associated with heart failure, liver failure or kidney failure in man or other mammals.

A further use for compounds of formula (I) which mimic the uterine smooth muscle effects of $PGE_2$ and $PGF_2$ is as anti-fertility agents, in particular as abortifacients.

In addition the compounds of formula (I) may be used in the treatment of proliferative skin diseases such as are associated with excessive cell division in the epidermis or dermis which may be accompanied by incomplete cell differentiation. Particular conditions which may be alleviated include

psoriasis, atopic dermatitis, non-specific dermatitis, primary irritant contact dermatitis, allergic contact dermatitis, basal and squamous cell carcin omas of the skin, lamellar ichthyosis, epidermolytic hyperkeratosis, premalignant sun induced keratosis, non malignat keratosis, acne and seborrheic dermatitis in humans and atopic dermatitis and mange in domestic animals. For the treatment of these conditions the compounds are desirably applied topically to the affected skin. Alternatively they may be administered by intradermal or intramuscular injection which may be directly into the skin lesion or into the surrounding tissue. Injectable compositions will generally contain from 0,1 to 0,5% w/v of active ingredient.

The amount of a compound of formula (I) required to achieve the desired biological effect will of course depend on a number of factors, for example, the specific compound chosen, the use for which it is intended, the mode of administration, and the recipient. In general, a daily dose may be expected to lie in the range of from 1 μg to 20 mg per kilogram bodyweight. Preferably the daily dose is 10 micro g to 2 mg, especially 100 micro g to 0,2 mg (200 micro g), per kilogram bodyweight. For example, an intravenous dose may lie in the range of from 5 μg to 1 mg/kg, preferably 50 μg to 100 μg/kg, which may conveniently be administered as an infusion of from 0,01 to 50 μg and preferably 0,1 to 5 μg, especially 0,5 to 1 μg per kilogram per minute. Infusion fluids suitable for this purpose may contain from 0,001 to 100, for example from 0,01 to 10 μg per millilitre. Unit doses may contain from 10 μg to 100 mg of a compound of formula (I), depending on how the compound is to be administered, for example ampoules for injection may contain from 0,01 to 1 mg, preferably 0,05 to 0,15 mg, for example 0,1 mg, and orally administrable unit dose formulations such as tablets or capsules may contain from 0,1 to 50, preferably 2 to 20 mg, especially 5 to 15 mg, for example 10 mg.

More specifically, when a compound of formula (I) is used to inhibit platelet aggregation it is generally desirable to achieve a concentration in the appropriate liquid, whether it be the blood of a patient or a perfusion fluid, of about 1 μg to 10 mg, preferably from 10 μg to 1 mg, especially 0,05 to 0,15 mg, for example 0,1 mg, per litre.

The abovementioned doses refer to the acids, amides, esters, alcohols and tetrazoles of formula (I); where a salt is used, the dose should be taken as referring to the corresponding anion.

For use in the treatment or prophylaxis of the conditions referred to above, while the hydantoin compounds may be used as the raw chemical they are preferably presented with an acceptable carrier therefor as a pharmaceutical formulation. The carrier must of course be 'acceptable' in the sense of being compatible with the other ingredients of the formulation and not deleterious to the recipient thereof. The carrier may be a solid or a liquid, and is preferably formulated with a hydantoin compound as a unit-dose formulation, for example a tablet, which may contain from 0,05% to 95% by weight of the hydantoin compound. Other pharmacologically active substances may also be present in formulations of the present invention as indicated above. The hydantoin compounds may be incorporated in the formulations either in the form of the acid or the salt, ester (or amide) thereof, and the formulations may be prepared by any of the well-known techniques of pharmacy consisting essentially of admixture of the components of the formulation.

The formulations includes those suitable for oral, rectal, topical (buccal — eg. sub-lingual), parenteral (that is subcutaneous, intramuscular and intravenous) administration, although the most suitable route in any give case will depend on the nature and severity of the condition being treated, and on the nature of the hydantoin compound.

Formulations suitable for oral administration may be presented as discrete units such as capsules, cachets, lozenges or tablets each containing a predetermined amount of hydantoin compound; as a powder or granules; as a solution or a suspension in an aqueous liquid or a non-aqueous liquid; as an oil-in-water emulsion; or as a water-in-oil liquid emulsion. Such formulations may be prepared by any of the methods of pharmacy but all methods include the step of bringing into association the hydantoin compound with the carrier which constitutes one or more accessory ingredients. In general they are prepared by uniformly and intimately admixing the hydantoin compound with liquid or finely divided solid carriers or both, and then, if necessary, shaping the product into the desired presentation. For example a tablet may be prepared by compression or moulding a powder or granules of the hydantoin compound, optionally with one or more accessory ingredients. Compressed tablets may be prepared by compressing, in suitable machine, the hydantoin compound in a free-flowing form such as a powder or granules optionally mixed with a binder, lubricant, inert diluent, surface active or dispersing agent(s). Moulded tablets may be made by moulding in a suitable machine the powdered hydantoin compound moistened with an inert liquid diluent.

Formulations suitable for buccal (sub-lingual) administration include lozenges comprising a hydantoin compound in a flavoured basis, usually sucrose and acacia or tragacanth; and pastilles comprising a hydantoin compound in an inert basis such as gelatin and glycerin; or sucrose and acacia.

Formulations suitable for topical application to the skin preferably take the form of an ointment, cream, lotion, paste, gel, spray, aerosol, or oil. Carriers which may be used include Vaseline® (trademark), lanoline, polyethylene glycols, alcohols and combinations thereof. The active ingredient is generally present in a concentration of from 0,1 to 15% w/w of the composition, for example from about 0,5 to about 2%.

Formulations of the present invention suitable for parenteral administration coveniently comprise

sterile aqueous preparations of a hydantoin compound, which preparations are preferably isotonic with the blood of the intended recipient. These preparations are preferably administered intravenously, although administration may also be effected by means of subcutaneous or intramuscular injection. Such preparations may be conveniently prepared by admixing the hydantoin compound with water and rendering the product sterile and isotonic with the blood.

Formulations suitable for rectal administration are preferably presented as unit-dose suppositories. These may be prepared by admixture of the hydantoin compound with one or more of the conventional solid carriers, for example cocoa butter, and shaping of the resulting mixture.

The invention is illustrated by the following examples which should not be construed as a limitation thereof.

Example 1

5-(6-Carboxyhexyl)-1-(3-hydroxyoctyl)-2-thiohydantoin

Diethyl 2-[(3-hydroxyoctyl)amino]nonanedioate (26,6 g) was stirred with 2N hydrochloric acid (28 ml) at room temperature for 1 hour and the water was evaporated *in vacuo* to leave the desired hydrochloride salt.

A stirred mixture of diethyl 2-[(3-hydroxyoctyl)amino]-nonanedioate hydrochloride (29,0 g) with ammonium thiocyanate (5,6 g) was heated at 158° for 1,25 hours. The cooled reaction mixture was treated with water (200 ml), the product was extracted into chloroform (4 x 30 ml), and the chloroform solution was washed with water and dried over magnesium sulphate. Evaporation of the chloroform yielded the cured 2-thiohydantoin ester. This ester (29,0 g) and 1N sodium hydroxide solution (130 ml) were stirred together at room temperature for 3 hours and neutral material was then removed with ether (2 x 40 ml). The aqueous alkaline phase was acidified with hydrochloric acid to pH 3, the liberated carboxylic acid was extracted into ether (4 x 50 ml) and the ethereal solution was washed with water and dried over magnesium sulphate. Evaporation of the ether yielded 5-(6-carboxyhexyl)-1-(3-hydroxyoctyl)-2-thiohydantoin as a mixture of 2 diastereomers which were separated by h.p.l.c. on $SiO_2$ in $CHCl_2$/MeOH/AcOH, 97,7 : 1,8 : 0,5. The individual diastereomers had relative $r_f$ values of 0,61 (more polar isomer) and 0,64 (less polar isomer) ($SiO_2$, $CHCl_3$/MeOH/HOAc, 90 : 5 :5).

Example 2 to 8

By the method of Example 1 the following compounds:

diethyl 2-[(5,5-dimethyl-3-hydroxyhexyl)amino]nonanedioate;

diethyl 2-[(3-cyclohexyl-3-hydroxypropyl)amino]nonanedioate;

diethyl 2-[(3-(4-*cis*-methylcyclohexyl)-3-hydroxypropyl)amino]nonanedioate;

diethyl 2-[(3-cyclopentyl-3-hydroxypropyl)amino]nonanedioate;

diethyl 2-[(3-cyclohexyl-3-hydroxypropyl)amino]non-4-enedioate;

diethyl 2-[(3-cyclopentyl-3-hydroxypropyl)amino]non-4-enedioate; and

diethyl 2-[(5,5-dimethyl-3-hydroxyhexyl)amino]non-4-enedioate;

were convered to the desired end products:

5-(6-carboxyhexyl)-1-(5,5-dimethyl-3-hydroxyhexyl)-2-thiohydantoin, which was separated into two diastereomers one a colorless gum, the other a white crystalline solid m.p. 123—126°C;

5-(6-carboxyhexyl)-1-(3-cyclohexyl-3-hydroxypropyl)-2-thiohydantoin which was separated into two diastereomers each of which was a colorless gum;

5-(6-carboxyhexyl)-1-(3-(4-*cis*-methylcyclohexyl)-3-hydroxypropyl)-2-thiohydantoin which was separated into two diastereomers each of which was a colourless gum;

5-(6-carboxyhexyl)-1-(3-cyclopentyl-3-hydroxypropyl)-2-thiohydantoin;

5-(6-carboxyhex-2*Z*enyl)-1-(3-cyclohexyl-3-hydroxypropyl)-2-thiohydantoin which was separated into diastereomers one a colourless and the other a white crystalline solid m.p. 109—111°C;

5-(6-carboxyhex-2*Z*enyl)-1-(3-cyclopentyl-3-hydroxypropyl)-2-thiohydantoin; and

5-(6-carboxyhex-2Z-enyl)-1-(5,5-dimethyl-3-hydroxyhexyl)-2-thiohydantoin;

which was separated into two diastereomers, one an oild and the other a crystalline solid m.p. 102—105°C.

Example 9

5-(6-carboxyhexyl)-3-methyl-1-octyl-2-thiohydantoin

A solution of diethyl 2-octylamino azelate (7,44 g) and methyl *iso* thiocyanate (1,15 g) in dry ether (21 ml) was allowed to stand at room temperature for 48 hours, after which time the ether was evaporated to leave an oil which was hydrolysed to the desired hydantoin using 5N sodium hydroxide solution for 3 hours at room temperature. The product, a viscous pale yellow oil, was obtained by extraction from ether and purified by chromatography on a column of silica gel.

Example 10

By a method analogous to that described in Example 9, but using ethyl 2-(6-ethoxycarbonylhexylamino)decanoate as starting material, there was obtained 1-(6-carboxyhexyl)-3-methyl-5-octyl-2-thiohydantoin as a pale yellow oil.

## Example 11

Interconversion of diastereomers

A solution of the hydantoin diastereomer to be converted is prepared in *N*-sodium hydroxide solution and allowed to stand at room temperature for several hours. The solution is then acidified and extracted with ether, and the ether extract is washed with water, dried and evaporated.

By means of high performance liquid chromatography the product remaining may be separated into two diastereomers; one identical with the starting material and the other being the other (second) diastereomer.

In similar fashion, the second diastereomer may converted into a mixture of approximately equal quantities of itself with the first diastereomer, and the pure diastereomers isolated by means of high performance liquid chromatography.

## Example 12

Inhibition of Platelet Aggregation

Aggregation of platelets in 1 ml of fresh human platelet rich plasma (PRP) was monitored in a Born aggregometer.

The compound to be tested was added to the PRP at the desired concentration, and the resulting mixture incubated at 37°C for 1 minute after which platelet aggregation was stimulated by the addition of adenosine diphosphate (ADP) to a concentration of 5 $\mu$m.

The antiaggregatory effect of the compound was assessed by measuring the percentage inhibition of platelet aggregation in the presence of the compound as compared with when it was absent. The percentage inhibitions at various concentrations of hydantoin and prostaglandin $E_1$ ($PGE_1$) were established and compared to show activity compared to $PGE_1$.

| Compound of Example No. | more or less polar diastereomer | Inhibition of Platelet Aggregation (X $PGE_1$) |
|---|---|---|
| 3 | less | 0,025 |
| | more | 0,008 |
| 2 | less | 0,04 |
| | more | 0,01 |
| 6 | less | 0,04 |
| | more | 0,02 |
| 5 | less | 0,03 |
| | more | 0,008 |
| 1 | less | <0,01 |
| | more | <0,01 |

## Example 13

Cardiovascular effects in rats

Male normotensive rats Wistar (Charles River) strain, (250—350 g) were anesthetised (chloroform) prior to cannulation to left femoral vein and anaestesia maintained by intravenous chloralose (60 mg/kg). Pulsatile blood pressure was recorded from the left femoral artery with an electronic transducer (Bell and Howell Type 4—327 L221) and integrated heart rate was measured with a cardiotachometer triggered from the arterial pressure waves.

The test compound (less polar diastereomer of the compounds of Example 2) was administered as a solution in physiological saline by intravenous injection *via* the femoral cannula. The responses recorded were allowed to return to the preinjection levels between successive administrations.

Injections of the vehicle alone or in volumes equivalent to those containing drug did not produce hypotension.

The test compound showed less than one per cent of the hypotensive effect of prostacyclin.

Example 14

| Tablet Formulation | In one tablet |
|---|---|
| Compound (less polar diastereomer of Example 3) | 10,0 mg |
| Microcrystalline cellulose B.P. | 200,0 mg |
| Starch B.P. | 15,0 mg |
| Magnesium Stearate | 1,0 mg |

The compound was dissolved in a volatile solvent. (The compound is soluble in methanol and in ethanol.) The solution was then evenly distributed over the microcrystalline cellulose, and then blended with the starch and then with the magnesium stearate. The mixture was then pressed to tablets, each 226 mg in weight.

Example 15

| Capsule Formulation | In one capsule |
|---|---|
| Compound as used in Example 14 | 10 mg |
| Polyethylene glycol 4000 | 190 mg |
| Magnesium Stearate | 1 mg |

The polyethylene glycol was melted and the compound was stirred in, and the mixture was cooled to room temperature. The wax produced was ground to give granules, which were mixed with the magnesium stearate and then filled into hard gelatine capsules containing 201 mg of mixture.

Example 16

| I μg/ml Injection | |
|---|---|
| Compound (as used in Example 14) | 100 μg |
| Water for Injection to | 100 ml |

The compound was dissolved in Water for Injection.

The solution was sterilised by filtration through a membrane filter, 0,22 μm pore size, collecting the filtrate in a sterile receiver. Under aseptic conditions, the solution was filled into sterile glass ampoules, 1 ml ampoule; The ampoule has sealed by fusion of the glass.

Example 17

| ı0 μg/ml Injection | |
|---|---|
| Compound (as used in Example 14) | 1 mg |
| Ethyl Alcohol | 10 ml |
| Propylene Glycol | 30 ml |
| Water for Injection to | 100 ml |

The compound was dissolved in the Ethyl Alcohol, the Propylene glycol was added and the mixture was diluted to volume with Water for Injection.

The solution was sterilised by filtration through a membrane filter, 0,22 μm pore size, collecting the filtrate in a sterile vessel. Under aseptic conditions, the solution was filled into sterile glass vials, 10 ml per vial. The vials were closed with a sterile rubber plug and secured with an aluminium collar.

Example 18

| 100 $\mu$g Single dose injection (freeze-dried) | |
|---|---|
| Compound (as used in Example 14) | 10,0 mg |
| Mannitol | 2,5 g |
| N/10 Sodium Hydroxide Solution qs to pH | 10,0 |
| Water for Injection to | 100,0 ml |

The compound was suspended in approximately 20 ml Water. Sufficient Sodium Hydroxide Solution was added to produce pH 10 and it was stirred to dissolve the Compound. The Mannitol was added and dissolved and the solution was diluted to volume with Water for Injection.

The solution was sterilised by passage through a membrane filter, 0,22 $\mu$m pore size, and distributed aseptically into sterile vials, 1 ml per vial. The solutions were freeze dried and the containers were sealed under aseptic conditions with rubber closures. Each vial contained 100 $\mu$g of Compound as its freeze-dried sodium salt.

Example 19

| Suppository Formulation | |
|---|---|
| Compound (as in Example 14) | 3 mg |
| Massa Esterinum C to | 2 g |

The suppository base was melted at about 40°C. Gradually the Compound was incorporated and mixed until homogenous. The melt was poured into suitable moulds and allowed to set.

Massa Esterinum C is a commercially available suppository base consisting of a mixture of mono-, di- and triglycerides of saturated vegetable fatty acids. It is marketed by Henkel International, Düsseldorf.

Example 20

| Soft Gelatine Capsule Formation | |
|---|---|
| Compound (as used in Example 14) | 10 mg |
| Vehicle about | 100 mg |

The Compound was diluted into a suitable vehicle which dissolved the compound and was then filtered into soft gelatine capsules, each containing about 110 mg of mixture.

Example 21

By the method of Example 1 diethyl 2-(3-hydroxybutyl)amino nonanedioate was converted to 5-(6-carboxyhexyl)-1-(3-hydroxybutyl)-2-thiohydantoin, two diastereomers: one- less polar- white crystalline solid, m.p. 92—95°C and the other- more polar- white crystalline solid, m.p. 102—104°C.

**Claims**

1. A compound of formula (I)

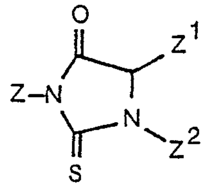

(I)

in which Z is hydrogen or alkyl having from 1 to 6 carbon atoms;

one of $Z^1$ and $Z^2$ is represented by the group $—CH_2—X—X^1—X^2$ wherein X is phenylene, $—C\equiv C—$, *cis* or *trans* $—CH=CH—$ or $—CH_2—CQ_2—$ in which each Q is independently selected from hydrogen and alkyl having from 1 to 6 carbon atoms or the two Q's together form an alkylene radical having four, five or six carbon atoms;

$X^1$ is a covalent bond or a straight or branched alkylene chain having from 1 to 6 carbon atoms, optionally having one methylene group replaced by an oxa (—O—) or thia (—S—) group, provided that at least one carbon atom separates such an oxa or thia group from a —C≡C—, —CH=CH— or —CO— group; and

$X^2$ is selected from 5-tetrazolyl, carboxyl, carbamoyl, hydroxymethyl, protected-hydroxymethyl and alkoxycarbonyl, wherein the alkyl group has from 1 to 6 carbon atoms and the other of $Z^1$ and $Z^2$ is represented by the group —Y—$Y^1$—$Y^2$—$Y^3$, wherein Y is —$CR_2$—$CH_2$, in which each R is independently selected from hydrogen and methyl;

$Y^1$ is carbonyl, methylene, methylene substituted by hydroxy or protected-hydroxy, or methylene substituted by hydroxy or protected hydroxy and by alkyl having from 1 to 6 carbon atoms;

Provided always that when $X^1$ is or includes an oxa or thia group than $Y^1$ is carbonyl, methylene substituted by hydroxy or protected-hydroxy, or methylene substituted by hydroxy or protected-hydroxy and by alkyl having from 1 to 6 carbon atoms;

$Y^2$ is a covalent bond or a straight or branched alkylene chain having from 1 to 7 carbon atoms, optionally substituted on the carbon atom adjacent $Y^1$ by one or two groups each of which may be alkyl having from 1 to 6 carbon atoms or a cyclic radical, $Y^3$ is hydrogen, hydroxy, protected-hydroxy, alkoxy having from 1 to 7 carbon atoms, a cyclic radical, phenyl, benzyl, phenoxy or benzoyloxy, wherein each of phenyl, benzyl, phenoxy and benzyloxy may be substituted in the benzene ring by one or more groups selected from hydroxy, protected-hydroxy, halo, nitro, amino, acylamino having from 1 to 6 carbon atoms, alkenyl having from 2 to 4 carbon atoms, alkoxy having from 1 to 6 carbon atoms, phenyl and alkyl having from 1 to 6 carbon atoms, which may itself be substituted by one or more halo groups; the cyclic radical in the definitions of $Y^2$ and $Y^3$ above being a radical derived by removal of a ring hydrogen atom from a monocyclic or polycyclic compound, other than benzene or naphthalene, having from 3 to 12 ring atoms selected from carbon, nitrogen, oxygen, and sulphur, which compound may be saturated or unsaturated and may be further substituted by one or more alkyl or fluoro groups, said alkyl groups having each from 1 to 6 carbon atoms;

or $Y^2$ and $Y^3$ together form an alkyl group, having from 1 to 7 carbon atoms, at least one hydrogen of which is replaced by fluoro;

or Y is a covalent bond, —$CH_2$—, or —$CH_2$—$CH_2$— and $Y^1$, $Y^2$ and $Y^3$ taken together form a cycloalkyl or bi-cycloalkyl group substituted by a hydroxy or protected-hydroxy group;

or a salt thereof.

2. A compound according to claim 1 in which Z is hydrogen or alkyl having from 1 to 4 carbon atoms;

X is —$CH_2$—$CH_2$—;

$X^1$ is alkylene having from 1 to 5 carbon atoms;

$X^2$ is carboxyl or alkoxycarbonyl, wherein the alkyl group has from 1 to 6 carbon atoms; and

$Y^3$ is hydrogen, phenyl, benzyl or cycloalkyl having from 4 to 7 atoms;

or a salt thereof.

3. A compound according to claim 1 or claim 2 in which Z is hydrogen and $Z^1$ is —$CH_2$—X—$X^1$—$X^2$ as defined as in claim 1 or claim 2.

4. A compound according to any preceding claim in which Y is —$CH_2$—$CH_2$—;

$Y^1$ is methylene substituted by hydroxy; and

$Y^2$ is a covalent bond or a branched alkylene chain having from 1 to 7 carbon atoms substituted on the carbon atom adjacent $Y^1$ by one or two alkyl groups having from 1 to 6 carbon atoms.

5. A compound according to any preceding claim in which $X^1$ is alkylene having 3 carbon atoms.

6. A compound according to any preceding claim in which $Y^3$ is cycloalkyl having from 4 to 7 carbon atoms.

7. The less polar diastereomer of a compound according to any preceding claim.

8. A compound according to claim 1 in which at least one of X to $X^2$ and Y to $Y^3$ has one of the values ascribed to it below;

(a) X is other than —$CH_2$—$CH_2$— or —CH=CH—; and/or

(b) $X^1$ is a covalent bond or a straight or branched hexylene chain or a straight or branched alkylene having from 1 to 6 carbon atoms having one methylene group replaced by an oxa or thia group;

(c) $X^2$ is 5-tetrazolyl, carbamoyl, hydroxymethylene or protected-hydroxymethylene; and/or

(d) Y is other than —$CH_2$—$CH_2$; and/or

(e) $Y^1$ is carbonyl; and/or

(f) $Y^2$ is alkylene having from 1 to 7 carbon atoms substituted on the carbon atom adjacent $Y^1$ by one or two groups each of which may be alkyl having from 1 to 6 carbon atoms or a cyclic radical as defined in claim 1, other than mono- or dimethyl substituted; and/or

(g) (i) when $Y^2$ is a covalent bond, then $Y^3$ is hydroxy or protected-hydroxy (except when $Y^1$ is methylene), or alkoxy having from 1 to 7 carbon atoms, a cyclic radical other than cycloalkyl having from 5 to 8 carbon atoms, phenoxy, or benzyloxy, or phenyl, benzyl, phenoxy or benzyloxy each of which is substituted in the benzene ring by one or more groups at least one of which is selected from amino, acylamino having from 1 to 6 carbon atoms, alkenyl

having from 2 to 4 carbon atoms, or alkyl having from 1 to 6 carbon atoms, substituted by one or more halogeno groups other than trifluoromethyl;

(g) (ii) when $Y^2$ is unsubstituted alkylene then $Y^3$ is as defined in claim 1 other than hydrogen or cycloalkyl having from 5 to 8 carbon atoms; and/or

(h) $Y^2$ and $Y^3$ together form an alkyl group, having from 1 to 7 carbon atoms, at least one hydrogen of which is replaced by fluoro; and/or

(i) Y is a covalent bond, $-CH_2-$ or $-CH_2-CH_2-$ and $Y^1$, $Y^2$ and $Y^3$ taken together form a cycloalkyl or bicycloalkyl group substituted by a hydroxy or protected-hydroxy group.

9. A compound according to any of the preceding claims for use in the treatment or prophylaxis of a thrombo-embolic disorder in a mammal.

10. A pharmaceutical formulation comprising a compound according to any one of the preceding claims together with a pharmaceutically acceptable carrier therefor.

11. A process for preparing a compound according to any one of claims 1 to 8 characterized by reacting a compound of formula (II),

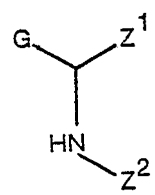
(II)

wherein $Z^1$ and $Z^2$ are as defined for formula (I), and G is a carboxyl, alkoxycarbonyl, carbamoyl or nitrile group; with thiocyanic acid, an alkyl*iso*thiocyanate, thiourea, nitrothiourea, or an *N*-alkylthiourea.

12. A process for preparing a compound according to any one of claims 1 to 8 wherein $X^2$ in formula (I) is 5-tetrazolyl characterized by reacting a compound of formula (I) as defined in claims 1 to 8 but in which $X^2$ is defined as:

$$-\underset{X^4}{\underset{|}{C}}=\underset{X^3}{\underset{|}{N}}$$

in which $X^3$ and $X^4$ together form a bond; or

$X^3$ is hydrogen or alkyl and $X^4$ is alkoxy, alkylthio, $-NH{\cdot}NH_2$, or amino; or

$X^3$ is hydroxy and $X^4$ is amino, with nitrous acid or hydrazoic acid or a salt thereof, as appropriate to form a compound of formula (I) in which $X^2$ is 5-tetrazolyl.

13. A process for preparing a compound according to any one of claims 1 to 8 wherein $X^2$ in formula (I) is hydroxymethylene, and/or $Y^1$ in formula (I) is a $-CHOH-$ group, characterised by reducing a compound of formula (I) (as defined in claims 1 to 8) in which $X^2$, as just defined is replaced by the corresponding acid, ester, acid halide, acid anhydride or aldehyde and/or $Y^1$ is a carbonyl group.

14. A process for preparing a compound according to any one of claims 1 to 8 wherein the compound of formula (I) contains a hydroxyl group, characterized by hydrolysing a compound of formula (I) (as defined in claims 1 to 8) in which the required hydroxy group is, in the starting material, represented by a halo group.

15. A process for preparing a compound according to any one of claims 1 to 8 wherein Y′ in formula (I) is a carbonyl group, characterized by oxidising a compound of formula (I) (as defined in claims 1 to 8) in which $Y^1$ is a $-CHOH-$ group.

16. A process for preparing a compound according to any one of claims 1 to 8 wherein X in formula (I) is a $-CH=CH-$ or a $-CH_2-CHQ-$ group (Q being as defined for formula (I) in claims 1 to 8) characterized by hydrogenating a compound of formula (I) (as defined in claims 1 to 8) in which X is $-C\equiv C-$ or $-CH=CQ-$.

**Patentansprüche**

1. Verbindung der Formel I

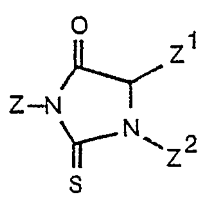
(I)

worin Z ein Wasserstoffatom oder einen Alkylrest mit 1 bis 6 Kohlenstoffatomen bedeutet, einer der Reste $Z^1$ und $Z^2$ durch die Gruppe $-CH_2-X-X^1-X^2$ dargestellt ist, worin X für Phenylen, $-C\equiv C-$, cis- oder trans-$CH=CH-$ oder $-CH_2-CQ_2-$steht,

worin jedes Q unabhängig unter Wasserstoff und Alkyl mit 1 bis 6 Kohlenstoffatomen ausgewählt ist oder beide Reste Q zusammen einen Alkylenrest mit vier, fünf oder sechs Kohlenstoffatomen bilden,

$X^1$ eine kovalente Bindung oder eine gerade oder verzweigte Alkylenkette mit 1 bis 6 Kohlenstoffatomen darstellt, worin gegebenenfalls eine Methylengruppe durch eine Oxa(—O—)- oder Thia(—S—)-Gruppe ersetzt ist, mit der Maßgabe, daß mindestens ein Kohlenstoffatom eine derartige Oxa- oder Thiagruppe von einer —C≡C—, —CH=CH— oder —CO—Gruppe trennt,

und $X^2$ unter 5-Tetrazolyl, Carboxyl, Carbamoyl, Hydroxymethyl, geschütztem Hydroxymethyl und Alkoxycarbonyl ausgewählt ist, worin der Alkylrest 1 bis 6 Kohlenstoffatome aufweist, und der andere Rest von $Z^1$ und $Z^2$ durch die Gruppe —Y—$Y^1$—$Y^2$—$Y^3$ angegeben ist, worin Y für —$CR_2$—$CH_2$— steht,

worin jedes R unabhängig unter Wasserstoff und Methyl ausgewählt ist,

$Y^1$ für Carbonyl, Methylen, durch eine Hydroxylgruppe oder durch eine geschützte Hydroxylgruppe substituiertes Methylen, oder durch eine Hydroxylgruppe oder geschützte Hydroxylgruppe und durch Alkyl mit 1 bis 6 Kohlenstoffatomen substituiertes Methylen steht,

immer mit der Maßgabe, daß sofern $X^1$ eine Oxa- oder Thiagruppe ist oder umfaßt, $Y^1$ für Carbonyl, durch Hydroxyl oder geschütztes Hydroxyl substituiertes Methylen, oder durch Hydroxyl oder geschütztes Hydroxyl und Alkyl mit 1 bis 6 Kohlenstoffatomen substituiertes Methylen steht,

$Y^2$ eine kovalente Bindung oder eine gerade oder verzweigte Alkylenkette mit 1 bis 7 Kohlenstoffatomen ist, die gegebenenfalls an dem Kohlenstoffatom, welches $Y^1$ benachbart ist, durch ein oder zwei Reste substituiert ist, die jeweils Alkyl mit 1 bis 6 Kohlenstoffatomen oder ein cyclischer Rest sind,

$Y^3$ für Wasserstoff, Hydroxyl, geschütztes Hydroxyl, Alkoxy mit 1 bis 7 Kohlenstoffatomen, einen cyclischen Rest, Phenyl, Benzyl, Phenoxy oder Benzoyloxy steht, worin jeder der Reste Phenyl, Benzyl, Phenoxy und Benzyloxy in dem Benzolring durch einen oder mehrere Reste von Hydroxyl, geschütztem Hydroxyl, Halogen, Nitro, Amino, Acylamino mit 1 bis 6 Kohlenstoffatomen, Alkenyl mit 2 bis 4 Kohlenstoffatomen, Alkoxy mit 1 bis 6 Kohlenstoffatomen, Phenyl mit 1 bis 6 Kohlenstoffatomen substituiert sein kann, wobei die Reste selbst durch ein oder mehrere Halogenatome substituiert sein können, wobei der cyclische Rest in den Definitionen von $Y^2$ und $Y^3$ ein Rest ist, der durch Entfernung eines Ring-Wasserstoffatoms von einer monocyclischen oder polycyclischen Verbindung, wobei Benzol und Naphthalin ausgenommen sind, mit 3 bis 12 Ringatomen, die unter Kohlenstoff, Stickstoff, Sauerstoff und Schwefel ausgewählt sind, abgeleitet wird,

wobei die Verbindung gesättigt oder ungesättigt sein kann und weiter durch einen oder mehrere Alkylreste mit jeweils 1 bis 6 Kohlenstoffatomen oder Fluoratome substituiert sein kann,

oder $Y^2$ und $Y^3$ zusammen einen Alkylrest mit 1 bis 7 Kohlenstoffatomen bilden, wovon mindestens ein Wasserstoffatom durch Fluor ersetzt ist,

oder Y eine kovalente Bindung, —$CH_2$— oder —$CH_2$—$CH_2$— darstellt und $Y^1$, $Y^2$ und $Y^3$ zusammen einen Cycloalkyl- oder Bicycloalkylrest bilden, der durch eine Hydroxylgruppe oder geschützte Hydroxylgruppe substituiert ist, oder ein Salz davon.

2. Verbindung nach Anspruch 1, worin Z ein Wasserstoffatom oder ein Alkylrest mit 1 bis 4 Kohlenstoffatomen ist,

X die Gruppe —$CH_2$—$CH_2$— darstellt,

$X^1$ einen Alkylenrest mit 1 bis 5 Kohlenstoffatomen bedeutet,

$X^2$ für Carboxyl oder Alkoxycarbonyl steht, worin der Alkylrest 1 bis 6 Kohlenstoffatome besitzt, und

$Y^3$ für Wasserstoff, Phenyl, Benzyl oder Cycloalkyl mit 4 bis 7 Kohlenstoffatomen steht, oder ein Salz davon.

3. Verbindung nach Anspruch 1 oder Anspruch 2, worin Z ein Wasserstoffatomen bedeutet und $Z^1$ den Rest —$CH_2$—X—$X^1$—$X^2$ gemäß der Definition in Anspruch 1 oder Anspruch 2 bedeutet.

4. Verbindung nach einem der voranstehenden Ansprüche, worin Y für —$CH_2$—$CH_2$— steht,

$Y^1$ durch eine Hydroxylgruppe substituiertes Methylen bedeutet, und

$Y^2$ eine kovalente Bindung oder eine verzweigte Alkylenkette mit 1 bis 7 Kohlenstoffatomen bedeutet, die an dem Kohlenstoffatom, welches dem Rest $Y^1$ benachbart ist, durch einen oder zwei Alkylreste mit 1 bis 6 Kohlenstoffatomen substituiert ist.

5. Verbindung nach einem der voranstehenden Ansprüche, worin $X^1$ ein Alkylenrest mit 3 Kohlenstoffatomen ist.

6. Verbindung nach einem der voranstehenden Ansprüche, worin $Y^3$ einen Cycloalkylrest mit 4 bis 7 Kohlenstoffatomen bedeutet.

7. Das weniger polare Diastereoisomere einer Verbindung nach einem der voranstehenden Ansprüche.

8. Verbindung nach Anspruch 1, worin mindestens einer der Reste X bis $X^2$ und Y bis $Y^3$ einen der nachfolgend beschriebenen Werte aufweist, worin

(a) X eine andere Bedeutung als —$CH_2$—$CH_2$— oder —CH=CH— aufweist, und/oder

(b) $X^1$ eine kovalente Bindung oder eine gerade oder verzweigte Hexylenkette oder einen geraden oder verzweigten Alkylenrest mit 1 bis 6 Kohlenstoffatomen bedeutet, in dem eine Methylengruppe durch eine Oxa- oder Thiagruppe ersetzt ist,

(c) $X^2$ für 5-Tetrazolyl, Carbamoyl, Hydroxymethylen oder geschütztes Hydroxymethylen steht, und/oder

(d) Y eine andere Bedeutung als $—CH_2—CH_2$ hat, und/oder

(e) $Y^1$ einen Carbonylrest darstellt, und/oder

(f) $Y^2$ einen Alkylenrest mit 1 bis 7 Kohlenstoffatomen darstellt, der an dem Kohlenstoffatom, welches $Y^1$ benachbart ist, durch einen oder zwei Reste substituiert ist, wovon jeder ein Alkylrest mit 1 bis 6 Kohlenstoffatomen oder ein cyclischer Rest gemäß der Definition in Anspruch 1 mit Ausnahme von Mono- oder Dimethyl-substituierten Resten sein kann, und/oder

(g) (i) sofern $Y^2$ eine kovalente Bindung ist, $Y^3$ eine Hydroxylgruppe oder eine geschützte Hydroxylgruppe (mit der Ausnahme, wenn $Y^1$ für Methylen steht) oder einen Alkoxyrest mit 1 bis 7 Kohlenstoffatomen, einen cyclischen Rest mit Ausnahme eines Cycloalkyls mit 5 bis 8 Kohlenstoffatomen, Phenoxy oder Benzyloxy, oder Phenyl, Benzyl, Phenoxy oder Benzyloxy, wovon jeder Rest am Benzolring durch eine oder mehrere Gruppen substituiert ist, wovon mindestens eine unter Amino, Acylamino mit 1 bis 6 Kohlenstoffatomen, Alkenyl mit 2 bis 4 Kohlenstoffatomen oder Alkyl mit 1 bis 6 Kohlenstoffatomen ausgewählt ist, die durch ein oder mehrere Halogenatome mit Ausnahme von Trifluormethyl substituiert sind, darstellt,

(g) (ii) sofern $Y^2$ unsubstituiertes Alkylen ist, $Y^3$ der Definition in Anspruch 1 entspricht, mit Ausnahme von Wasserstoff oder Cycloalkyl mit 5 bis 8 Kohlenstoffatomen, und/oder

(h) $Y^2$ und $Y^3$ zusammen einen Alkylrest bilden, der 1 bis 7 Kohlenstoffatome aufweist, wobei mindestens ein Wasserstoffatom durch ein Fluoratom ersetzt ist, und/oder

(i) Y eine kovalente Bindung, $—CH_2—$ oder $—CH_2—CH_2—$ ist und $Y^1$, $Y^2$ und $Y^3$ zusammen eine Cycloalkyl- oder Bicycloalkylrest bilden, der durch eine Hydroxylgruppe oder eine geschützte Hydroxylgruppe substituiert ist.

9. Verbindung nach einem der voranstehenden Ansprüche zur Verwendung bei der Behandlung oder Prophylaxe einer thrombo-embolischen Erkrankung eines Säugetiers.

10. Pharmazeutische Formulierung, die eine Verbindung nach einem der voranstehenden Ansprüche zusammen mit einem pharmazeutisch verträglichen Träger dafür enthält.

11. Verfahren zur Herstellung einer Verbindung nach einem der Ansprüche 1 bis 8, dadurch gekennzeichnet, daß eine Verbindung der Formel II

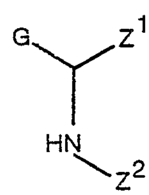

(II)

worin $Z^1$, und $Z^2$ der Definition für Formel I entsprechen und G ein Carboxyl-, Alkoxycarbonyl-, Carbamoyl- oder Nitrilrest ist, mit Thiocyansäure, einem Alkylisothiocyanat, Thioharnstoff, Nitrothioharnstoff oder einem N-Alkylthioharnstoff umgesetzt wird.

12. Verfahren zur Herstellung einer Verbindung nach einem der Ansprüche 1 bis 8, worin $X^2$ in Formel I für 5-Tetrazolyl steht, dadurch gekennzeichnet, daß eine Verbindung der Formel I gemäß der Definition in den Ansprüchen 1 bis 8, worin jedoch $X^2$ definiert ist als

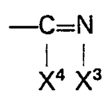

worin $X^3$ und $X^4$ zusammen eine Bindung bilden, oder $X^3$ ein Wasserstoffatom oder einen Alkylrest bedeutet und $X^4$ einen Alkoxy-, Alkylthio-, $—NH·NH_2$ oder Aminorest darstellt, oder

$X^3$ eine Hydroxylgruppe ist und $X^4$ eine Aminogruppe bedeutet,

mit salpetriger Säure oder Stickstoffwasserstoffsäure oder einem Salz davon, wie es geeignet ist zur Bildung einer Verbindung der Formel I, worin $X^2$ für 5-Tetrazolyl steht, umgesatzt wird.

13. Verfahren zur Herstellung einer Verbindung nach einem der Ansprüche 1 bis 8, worin $X^2$ in der Formel I für Hydroxymethylen steht und/oder $Y^1$ in Formel I eine —CHOH-Gruppe darstellt, dadurch gekennzeichnet, daß eine Verbindung der Formel I (gemäß der Definition in den Ansprüchen 1 bis 8), worin $X^2$ gemäß obiger Definition durch die entsprechende Säure, den Ester, Säurehalogenid, Säureanhydrid oder Aldehyd ersetzt wird und/oder $Y^1$ eine Carbonylgruppe ist, reduziert wird.

14. Verfahren zur Herstellung einer Verbindung nach einem der Ansprüche 1 bis 8, worin die Verbindung der Formel I eine Hydroxylgruppe enthält, dadurch gekennzeichnet, daß eine Verbindung der Formel I (gemäß der Definition in den Ansprüchen 1 bis 8), worin die benötigte Hydroxylgruppe in dem Ausgangsmaterial durch ein Halogenatom dargestellt ist, hydrolysiert wird.

15. Verfahren zur Herstellung einer Verbindung nach einem der Ansprüche 1 bis 8, worin Y' in

Formel I eine Carbonylgruppe bedeutet, dadurch gekennzeichnet, daß eine Verbindung der Formel I (gemäß der Definition in den Ansprüchen 1 bis 8), worin $Y^1$ für einen Rest —CHOH— steht, oxidiert wird.

16. Verfahren zur Herstellung einer Verbindung nach einem der Ansprüche 1 bis 8, worin X in Formel I ein Rest —CH = CH— oder ein Rest —$CH_2$—CHQ— ist (worin Q der Definition in Formel I in den Ansprüchen 1 bis 8 entspricht), dadurch gekennzeichnet, daß eine Verbindung der Formel I (gemäß der Definition in den Ansprüchen 1 bis 8), worin X für —C ≡ C— oder —CH = CQ— steht, hydriert wird.

**Revendications**

1. Composé de formule (I)

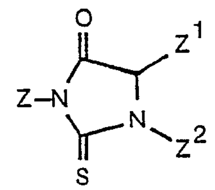

(I)

où Z représente un atome d'hydrogène ou un radical alcoyle de 1 à 6 atomes de carbone;

l'un d'entre $Z^1$ et $Z^2$ représente le radical —$CH_2$—X—$X^1$—$X^2$ où X représente un radical phénylène, —C ≡ C, *cis* ou *trans* —CH = CH— ou —$CH_2$—$CQ_2$— dans lequel chaque Q est choisi indépendamment entre un atome d'hydrogène et un radical alcoyle de 1 à 6 atomes de carbone ou les deux Q ensemble forment un radical alcoylène de 4, 5 ou 6 atomes de carbone;

$X^1$ représente une liaison covalente ou une chaîne alcoylène droite ou ramifiée de 1 à 6 atomes de carbone dans laquelle un radical méthylène est éventuellement remplacé par un radical oxa (—O—) ou thia (—S—), étant entendu qu'au moins un atome de carbone sépare un tel radical oxa ou thia d'un radical —C ≡ C—, —CH = CH— ou —CO— et

$X^2$ représente un radical 5-tétrazolyle, carboxyle, carbamoyle, hydroxyméthyle, hydroxyméthyle protégé ou alcoxycarbonyle dont le radical alcoyle compte 1 à 6 atomes de carbone, et l'autre d'entre $Z^1$ et $Z^2$ représente le radical —Y—$Y^1$—$Y^2$—$Y^3$, où représente un radical —$CR_2$—$CH_2$ dans lequel chaque R est choisi indépendamment entre un atome d'hydrogène et un radical méthyle;

$Y^1$ représente un radical carbonyle, méthylène, méthylène substitué par hydroxyle ou hydroxyle protégé ou méthylène substitué par hydroxyle ou hydroxyle protégé et par alcoyle de 1 à 6 atomes de carbone, *étant toujours entendu* que $X^1$ est ou comprend un radical oxa ou thia lorsque $Y^1$ représente un radical carbonyle, méthylène substitué par hydroxyle ou hydroxyle protégé ou méthylène substitué par hydroxyle ou hydroxyle protégé et par alcoyle de 1 à 6 atomes de carbone;

$Y^2$ représente une liaison covalente ou une chaîne alcoylène droite ou ramifié de 1 à 7 atomes de carbone éventuellement substituée sur l'atome de carbone adjacent à $Y^1$ par un ou deux radicaux dont chacun peut être un radical alcoyle de 1 à 6 atomes de carbone ou un radical cyclique,

$Y^3$ représente un atome d'hydrogène ou un radical hydroxyle, hydroxyle protégé, alcoxy de 1 à 7 atomes de carbone, un radical cyclique, phényle, benzyle, phénoxy ou benzyloxy, dont chaque radical phényle, benzyle, phénoxy ou benzyloxy peut être substitué sur le cycle benzénique par un ou plusieurs radicaux choisi parmi hydroxyle, hydroxyle protégé, halo, nitro, amino, acylamino de 1 à 6 atomes de carbone, alcényle de 2 à 4 atomes de carbone, alcoxy de 1 à 6 atomes de carbone, phényle et alcoyle de 1 à 6 atomes de carbone pouvant lui-même être substitué par un ou plusieurs radicaux halo;

le radical cyclique dans les définitions de $Y^2$ et $Y^3$ ci-dessus étant un radical issu par élimination d'un atome d'hydrogène de cycle d'un composé monocyclique ou polycyclique autre que le benzène ou le naphtalène, comptant 3 à 12 atomes de cycle choisis entre les atomes de carbone, d'azote, d'oxygène et de soufre, lequel composé peut être saturé ou insaturé et peut être en outre substitué par un plusieurs radicaux alcoyle ou fluoro, lesquels radicaux alcoyle comptent chacun 1 à 6 atomes de carbone;

ou bien $Y^2$ et $Y^3$ forment ensemble un radical alcoyle comptant 1 à 7 atomes de carbone dont au moins un atome d'hydrogène est remplacé par un radical fluoro;

ou bien Y représente une liaison covalente ou un radical —$CH_2$—, ou —$CH_2$—$CH_2$— et $Y^1$, $Y^2$ et $Y^3$ pris ensemble forment un radical cycloalcoyle ou bicyclo-alcoyle substitué par un radical hydroxyle ou hydroxyle protégé;

ou un sel de ce composé.

2. Composé suivant la revendication 1, dans lequel

Z représente un atome d'hydrogène ou un radical alcoyle de 1 à 4 atomes de carbone,

X représente —$CH_2$—$CH_2$—;

$X^1$ représente une chaîne alcoylène de 1 à 5 atomes de carbone;

$X^2$ représente un radical carboxyle ou alcoxycarbonyle dont le radical alcoyle compte 1 à 6 atomes de carbone, et

$Y^3$ représente un atome d'hydrogène ou un radical phényle, benzyle ou cycloalcoyle de 4 à 7 atomes de carbone;

ou un sel de ce composé.

3. Composé suivant la revendication 1 ou 2, dans lequel Z représente un atome d'hydrogène et $Z^1$ représente $—CH_2—X—X^1—X^2$ tel que défini dans la revendication 1 ou 2.

4. Composé suivant l'une quelconque des revendications précédentes, dans lequel Y représente $—CH_2—CH_2—$;

$Y^1$ représente un radical méthylène substitué par hydroxyle, et $Y^2$ représente une liaison covalente ou une chaîne alcoylène ramifiée de 1 à 7 atomes de carbone substitués sur l'atome de carbone adjacent à $Y^1$ par un ou deux radicaux alcoyle de 1 à atomes de carbone.

5. Composé suivant l'une quelconque des revendications précédentes, dans lequel

$X^1$ représente un chaîne alcoylène de 3 atomes de carbone.

6. Composé suivant l'une quelconque des revendications précédentes, dans lequel

$Y^3$ représente un radical cycloalcoyle de 4 à 7 atomes de carbone.

7. Le diastéréoisomère le moins polaire d'un composé suivant l'une quelconque des revendications précédentes.

8. Composé suivant la revendication 1, dans lequel au moins l'un d'entre X à $X^2$ et Y à $Y^3$ a l'une des significations qui lui est attribuée ci-après:

(a) X est autre que $—CH_2—CH_2—$ ou $—CH=CH—$, et/ou

(b) $X^1$ représente une liaison covalente ou une chaîne hexylène droite ou ramifiée ou une chaîne alcoylène droite ou ramifiée de 1 à 6 atomes de carbone dont un radical méthylène est remplacé par un radical oxa ou thia;

(c) $X^2$ représente un radical 5-tétrazolyle, carbamoyle, hydroxyméthylène ou hydroxyméthylène protégé, et/ou

(d) Y est autre que $—CH_2—CH_2—$, et/ou

(e) $Y^1$ représente un radical carbonyle, et/ou

(f) $Y^2$ représente une chaîne alcoylène de 1 à 7 atomes de carbone substituée sur l'atome de carbone adjacent à $Y^1$ par un ou deux radicaux dont chacun peut être un radical alcoyle de 1 à 6 atomes de carbone ou un radical cyclique tel que défini dans la revendication 1, autre que mono- ou diméthyl-substitué, et/ou

(g) (i) lorsque $Y^2$ représente une liaison covalente, alors $Y^3$ représente un radical hydroxyle ou hydroxyle protége (sauf lorsque $Y^1$ représente un radical méthylène), ou un radical alcoxy de 1 à 7 atomes de carbone, un radical cyclique autre que cycloalcoyle comptant 5 à 8 atomes de carbone, phénoxy ou benzyloxy ou bien phényle, benzyle, phénoxy ou benzyloxy dont chacun est substitué sur le cycle benzénique par un ou plusiers radicaux dont au moins un est choisi parmi les radicaux amino, alcylamino de 1 à 6 atomes de carbone, alcényle de 2 à 4 atomes de carbone et alcoyle de 1 à 6 atomes de carbone, substitué par un ou plusieurs radicaux halogéno autre que trifluorométhyle;

(g) (ii) lorsque $Y^2$ représente un chaîne alcoylène non substituée, alors $Y^3$ est tel que défini dans la revendication 1 à l'exception d'hydrogène ou cyclo-alcoyle comptant 5 à 8 atomes de carbone, et/ou

(h) $Y^2$ et $Y^3$ forment ensemble un radical alcoyle de 1 à 7, atomes de carbone dont au moins un atome d'hydrogène est remplacé par un radical fluoro, et/ou

(i) Y représente une liaison covalente ou un radical $—CH_2—$ ou $—CH_2—CH_2—$ et $Y^1$, $Y^2$ et $Y^3$ pris ensemble forment un radical cyclo-alcoyle ou bicyclo-alcoyle substitué par un radical hydroxyle ou hydroxyle protégé.

9. Composé suivant l'une quelconque des revendications précédentes, à utiliser pour le traitement ou la prophylaxie d'un trouble thrombo-embolique chez un mammifère.

10. Composition pharmaceutique comprenant un composé suivant l'une quelconque des revendications précédentes outre un excipient pharmaceutiquement acceptable.

11. Procédé de préparation d'un composé suivant l'une quelconque des revendications 1 à 8, caractérisé par la réaction d'un composé de formule (II)

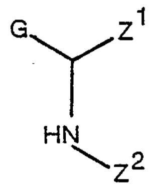

(II)

où $Z^1$ et $Z^2$ sont tels que définis à propos de la formule (I) et G représente un radical carboxyle, alcoxycarbonyle, carbamoyle ou nitrile, avec l'acide thiocyanique, un *iso*thiocyanate d'alcoyle, la thiourée, la nitrothiourée ou une *N*-alcoylthiourée.

12. Procédé de préparation d'un composé suivant l'une quelconque des revendications 1 à 8, où

$X^2$ dans la formule (I) représente un radical 5-tétrazolyle, caractérisé par la réaction d'un composé de formule (I) tel que défini dans les revendications 1 à 8, mais où $X^2$ est défini comme étant:

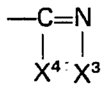

où $X^3$ et $X^4$ forment ensemble une liaison, ou

$X^3$ représente un atome d'hydrogène ou radical alcoyle et $X^4$ représente un radical alcoxy, alcoylthio, —NH.NH$_2$ ou amino, ou bien

$X^3$ représente un radical hydroxyle et $X^4$ représente un radical amino, avec l'acide nitreux ou l'acide hydrazoïque ou un sel de ceux-ci, suivant les besoins, pour former un composé de formule (I) où $X^2$ représente un radical 5-tétrazolyle.

13. Procédé de préparation d'un composé suivant l'une quelconque des revendications 1 à 8, où $X^2$ dans la formule (I) représente un radical hydroxyméthylène et/ou $Y^1$ dans la formule (I) représente un radical —CHOH—, caractérisé par la réduction d'un composé de formule (I) (tel que défini dans les revendications 1 à 8) où $X^2$ tel que défini immédiatement ci-dessus est remplacé par l'acide, eter, halogénure d'acide, anhydride d'acide ou aldéhyde correspondant et/ou $Y^1$ représente un radical carbonyle.

14. Procédé de préparation d'un composé suivant l'une quelconque des revendications 1 à 8, où le composé de formule (I) contient un radical hydroxyle, caractérisé par l'hydrolyse d'un composé de formule (I) (tel que défini dans les revendications 1 à 8) où la place du radical hydroxyle requis est occupée, dans le composé de départ, par un radical halo.

15. Procédé de préparation d'un composé suivant l'une quelconque des revendications 1 à 8, où Y' dans la formule (I) représente un radical carbonyle, caractérisé par l'oxydation d'un composé de formule (I) (tel que défini dans les revendications 1 à 8) où $Y^1$ représente un radical —CHOH—.

16. Procédé de préparation d'un composé suivant l'une quelconque des revendications 1 à 8, où X dans la formule (I) représente un radical —C = CH— ou —CH$_2$—CHQ— (Q étant tel que défini à propos de la formule (I) dans les revendications 1 à 8) caractérisé par l'hydrogénation d'un composé de formule (I) (tel que défini dans les revendications 1 à 8) où X représente —C ≡ C— ou —CH = CQ—.